# FASCICULE DE BREVET EUROPEEN

(11) **EP 0 747 047 B1**
(45) Date de publication et mention de la délivrance du brevet: **15.07.1998**
(21) Numéro de dépôt: 96400763.7
(22) Date de dépôt: 09.04.1996
(51) Int. Cl.: A61K 31/16, A61K 31/08

(54) **Association d'un composé à activité anti-microbienne (e.g. du lipoaminoacide N,n-Octanoyl-glycine) et d'un monoalkyléther de glycérol et son usage en tant d'agent anti-microbien**
Kombination einer antimikrobiellen Verbindung (z.B. der Lipoaminosäure N,n-Octanoyl-glycin) und eines Monoalkylglycerinethers und ihre Verwendung als antimikrobieller Wirkstoff
Combination of an antimicrobial compound (e.g. the lipoaminoacid N,n-octanoyl-glycine) and a monoalkylglycerol ether and its use as an antimicrobial agent

(30) Priorité: 17.05.1995 FR 9505866
(43) Date de publication de la demande: 11.12.1996
(73) Titulaire: LA ROCHE POSAY Laboratoire Pharmaceutique, 86270 La Roche Posay (FR)
(72) Inventeur: Burnier, Véronique, 86100 Chatellerault (FR); Brissonnet, Jean-Pierre, 86000 Poitiers (FR)
(74) Mandataire: Tezier Herman, Béatrice

(56) Documents cités:
- DE-A- 2 415 750
- DE-A- 4 140 474
- DE-C- 4 240 674
- FR-A- 2 411 006
- ANN. PHARM. FR., vol. 39, no. 6, 1981, pages 503-510, XP002010840 MARHNOUJ, L. ET AL: "Experimental study of bacteriostatic activity of some lipoamino acids and their derivatives"
- ANTIMICROB. AGENTS CHEMOTHER., 1994, 38/7 (1523-1529), USA, XP002010841 HAYNES M.P. ET AL: "Synergism between the antifungal agents amphotericin B and alkyl glycerol ethers"
- LIPIDS, 1990, vol. 25, no. 2, pages 119-121, XP002010842 VED, H. S. ET AL: "Synergism between penicillin G and the antimicrobial ether lipid, rac-1-dodecylglycerol, acting below its critical micelle concentration"
- J. BIOL. CHEM., 1986, vol. 261, no. 14, XP002010843 BRISSETTE, JANICE L. ET AL: "Studies on the antibacterial activity of dodecylglycerol. Its limited metabolism and inhibition of glycerolipid and lipoteichoic acid biosynthesis in Streptococcus mutans BHT"
- PROC. SOC. EXP. BIOL. MED., 1991, vol. 197, no. 1, pages 91-97, XP002010844 NGWENYA, BENJAMIN Z. ET AL: "Activation of peritoneal macrophages by orally administered ether analogs of lysophospholipids"

## Description

L'invention concerne une association synergique d'au moins un composé à activité anti-microbienne et d'au moins un monoalkyléther de glycérol, utilisée notamment en tant qu'agent anti-microbien dans une composition. Cette association trouve une application toute particulière dans les domaines cosmétiques ou pharmaceutiques.

Il est courant d'introduire dans les compositions cosmétiques ou dermatologiques des conservateurs chimiques destinés à lutter contre le développement des microorganismes dans ces compositions, qui les rendraient rapidement inaptes à l'utilisation. Il faut protéger les compositions à la fois contre les microorganismes susceptibles de se développer à l'intérieur de la composition et contre ceux que l'utilisateur peut introduire dans celle-ci en la manipulant, et en particulier lors de la préhension avec les doigts de produits en pot.

Des conservateurs chimiques couramment utilisés sont notamment les parabens ou des donneurs de formol. Ces conservateurs présentent l'inconvénient de causer des intolérances, telles que irritations et/ou allergies, et en particulier sur les peaux sensibles. Il en est de même pour les alcools ou les polyols, tels que l'éthanol ou le propylène glycol, en particulier lorsqu'ils sont présents à des taux relativement importants.

On constate donc que subsiste le besoin d'agents anti-microbiens ayant une action au moins aussi efficace que les composés de l'art antérieur, mais ne présentant pas leurs inconvénients.

Il est connu que des lipoaminoacides ou des lipides ont une certaine activité anti-microbienne, tel que décrit dans Ann. Pharm. FR., vol. 39, no.6, 1981, pages 503-510, Marhnouj, L. et al et les brevets DE 24 15 750 et FR 2 411 006. Cependant, cette activité peut s'avérer parfois insuffisante. S'ils sont présents à des taux relativement importants, ces lipoaminoacides ou ces lipides peuvent également provoquer des intolérances, plus particulièrement lorsqu'ils comprennent des acides gras à chaînes courtes.

Ainsi, la demanderesse a cherché à résoudre ces problèmes.

La demanderesse a, de manière surprenante, découvert qu'une association comprenant au moins un composé à activité anti-microbienne choisi parmi des hydrolipides ou des lipides et au moins un monoalkyléther de glycérol présente une synergie au niveau de l'activité anti-microbienne, plus particulièrement au niveau d'une bactérie gram -, la Pseudomonas aeruginosa. Les monoalkyléthers de glycérol, et plus particulièrement le dodécyl glycérol, sont décrits comme des agents antimicrobiens dans J. Biol. Chem., 1986, vol. 261, no. 14, Brissette, Janice et al et dans Proc. Soc. Exp. Biol. Med., 1991, vol. 197, no. 1, Pages 91-97, Ngwenya, Benjamin Z. et al, mais ils sont de faibles antifongiques et des antibactériens gram +.

Cette association est avantageuse, car elle ne provoque pas d'intolérance et/ou de rougeur lors de l'application sur la peau d'une composition cosmétique ou dermatologique comprenant cette association, tout en présentant une activité anti-microbienne au moins aussi efficace que celle des composés de l'art antérieur. Cette activité anti-microbienne est particulièrement intéressante puisqu'elle lutte plus particulièrement contre le développement d'un des contaminants les plus gênants et les plus courants dans le domaine cosmétique : les Pseudomonas aeruginosa.

Ainsi, la présente invention a pour objet une composition à usage topique comprenant au moins un composé à activité anti-microbienne choisi parmi des hydrolipides, qui sont des produits résultant du couplage d'acides gras avec des molécules hydrophiles choisies parmi les acides aminés et les polysaccharides, et des lipides, qui sont des acides gras ramifiés ou non, saturés ou insaturés, de 6 à 18, de préférence de 6 à 12 atomes de carbone ou qui sont choisis parmi l'acide myristique, myristoléique, palmitique, palmitoléique, oléique, linoléique et linolénique, et au moins un monoalkyléther de glycérol.

Un autre objet de l'invention est l'utilisation de cette composition en tant qu'agent anti-microbien dans une composition cosmétique ou pharmaceutique.

Cette composition est plus particulièrement destinée à un usage cosmétique ou pharmaceutique, de préférence dermatologique, et comprend donc un support cosmétiquement ou pharmaceutiquement acceptable.

Dans le cadre d'une utilisation pharmaceutique, la présente invention a également pour objet l'utilisation de cette composition en tant qu'agent anti-microbien dans la préparation d'une composition pharmaceutique, plus particulièrement dermatologique.

Par activité anti-microbienne, on entend selon l'invention une activité anti-bactérienne et/ou une activité anti-fongique et/ou une activité anti-virale.

Par hydrolipide, on entend selon l'invention des produits résultant du couplage d'acides gras avec des molécules hydrophiles choisies parmi les acides aminés (monomères, peptides ou protéines) et les polysaccharides. Ce couplage des deux composés peut résulter en des composés à liaisons covalentes, notamment entre les acides aminés et les acides gras (du type -CONH-), mais également en des composés à liaisons non covalentes. De préférence, on utilise des hydrolipides présentant uniquement des liaisons covalentes.

Les acides gras présents dans les hydrolipides sont ramifiés ou non, saturés ou insaturés. Ils présentent généralement de 6 à 18, de préférence de 6 à 12, atomes de carbone.

Lorsque les hydrolipides comprennent des acides aminés, ces derniers sont donc sous forme de monomères, de peptides ou de protéines. On préfère sous forme de monomères.

A titre d'exemples d'acides aminés, on peut citer la méthionine, la lysine, l'acide aspartique, la glycine et la cystéïne. De préférence, les acides aminés sont la glycine ou la cystéïne. On utilise plus particulièrement le N,n-octanoyl-glycine, vendu et commercialisé par SEPPIC sous la dénomination Lipacid ® C8G.

Les protéïnes peuvent être d'origine naturelle ou synthétique. Elles peuvent être présentes sous forme hydrolysée, on parle alors de peptides ou d'acides aminés.

Ainsi, parmi les protéines d'origine animale, on préfère : kératine, soie, collagène, réticuline, élastine, lactalbumine, lactoglobuline, caséine, ovalbumine. Parmi les protéines d'origine végétale, on préfère : les protéines de blé, de maîs, d'avoine, de soja et d'amande. Parmi les protéines d'origine marine, on préfère : collagène, protéines de poisson, de mollusques, de crustacés et d'algues.

Certains de ces hydrolipides, plus communément appelés lipoaminoacides, résultant de la réaction entre des acides gras et d'acides aminés sont notamment décrits dans les brevets FR 2192795, FR 2411006 et FR 2422400.

Lorsque les hydrolipides comprennent des polysaccharides, ces derniers peuvent être d'origine naturelle ou synthétique.

Ainsi, parmi les polysaccharides d'origine animale, on peut notamment citer l'acide hyaluronique, chondroïtine-4-sulfate, chondroïtine-6-sulfate, dermatane sulfate, héparane sulfate, kératane sulfate, l'héparine et ses dérivés.

Parmi les polysaccharides d'origine végétale, on peut notamment citer l'amylose, l'amylopectine, les glucomannanes, les galactomannanes, les fructosanes, les gommes, les celluloses et leurs dérivés.

Parmi les polysaccharides d'origine marine, on peut notamment citer la chondroïtine sulfate, le chitosan, les alginates, l'aguar et le carraghénane.

Lorque ces polysaccharides sont d'origine synthétique, ils peuvent être obtenus par fermentation. On peut ainsi citer, à titre d'exemples : xanthane, gellane, curdlane et dextrane.

A titre d'hydrolipides dans le commerce, on peut également citer les produits de la famille des "Lifidrem®" commercialisés par la société COLETICA, tels que les Lifidrem® Coun (molécule hydrophile : collagène, acide gras : acide undécylénique), Blun (molécule hydrophile : protéines de blé, acide gras : acide undécylénique), Avun (molécule hydrophile : protéines d'avoine, acide gras : acide undécylénique), amun (molécule hydrophile : protéines d'amande douce, acide gras : acide undécylénique), Syun (molécule hydrophile : protéines de soja, acide gras : acide undécylénique), Arun (molécule hydrophile : protéines d'algues roses, acide gras : acide undécylénique), Arca (molécule hydrophile : protéines d'algues roses, acide gras : acide caprylique).

Par lipide, on entend selon l'invention des acides gras ramifiés ou non, saturés ou insaturés présentant de 6 à 12 atomes de carbone ou des acides gras choisis parmi l'acide myristique, myristoléique, palmitique, palmitoléique, oléique, linoléique, linolénique, et undécylénique. On préfère l'acide caprylique, caprique, laurique et undécylénique.

On préfère selon l'invention utiliser les hydrolipides et plus particulièrement les lipoaminoacides.

Les monoalkyléthers de glycérol utilisés présentent généralement la formule (I) suivante:

R-O-CH₂-CHOH-CH₂OH (I)

dans laquelle R représente un radical alkyle linéaire ou ramifié, saturé ou insaturé, de 3 à 14 atomes de carbone, en particulier de 5 à 12 atomes de carbone, de préférence de 7 à 9 atomes de carbone.

Parmi ces composés, on peut notamment citer le 1-heptylglycéroléther, le 1-(2-ethylhexyl)glycéroléther, le 1-octylglycéroléther, le 1-décylglycéroléther et le 1-dodécylglycéroléther. Parmi ceux-ci, on préfère utiliser le 1-(2-ethylhexyl)glycéroléther.

Le rapport pondéral du composé à activité anti-microbienne par rapport au monoalkyléther de glycérol peut varier dans une large mesure. Il est de préférence compris entre 0,05 et 30, plus particulièrement entre 0,1 et 5.

Plus particulièrement, la composition cosmétique ou pharmaceutique selon l'invention est appliquée par voie topique.

Cette composition synergique présente donc notamment l'avantage que le composé à activité anti-microbienne peut être présent dans une composition à des quantités plus faibles que lorsqu'il est utilisé seul.

La composition comprend préférentiellement de 0,01 à 6 %, avantageusement de 0,1 à 2 %, en poids de monoalkyléther de glycérol par rapport au poids total de la composition.

La composition comprend préférentiellement de 0,01 à 5 %, avantageusement de 0,2 à 3 %, en poids de composé à activité anti-microbienne par rapport au poids total de la composition.

Les compositions de l'invention, dans le cas d'une application cosmétique ou dermatologique, peuvent donc comprendre en outre les adjuvants cosmétiques ou pharmaceutiques classiquement mis en oeuvre comme les corps gras, les solvants organiques, les épaississants ioniques ou non ioniques, les adoucissants, les antioxydants, les opacifiants, les stabilisants, les silicones, les agents anti-mousse, les agents hydratants, les vitamines, les parfums, les tensioactifs ioniques ou non, les charges, les séquestrants, les colorants ou tout autre ingrédient habituellement utilisé en cosmétique ou dermatologie.

Les compositions selon l'invention sont préparées selon les techniques bien connues de l'homme de l'art du domaine.

Les compositions selon l'invention peuvent se présenter sous forme de solution, de suspension ou de dispersion dans des solvants ou des corps gras, sous forme de dispersion vésiculaire ou encore sous forme d'émulsion telle qu'une crème ou un lait, sous forme de pommade, de gel, de bâtonnet solide, de mousse aérosol ou de spray.

Dans tout ce qui suit ou ce qui précède, les pourcentages sont donnés en poids sauf mention contraire.

D'autre caractéristiques et avantages de l'invention pourront apparaître dans les exemples qui suivent, qui sont donnés à titre purement illustratif et nullement limitatif.

### EXEMPLE 1

Les tests suivants mettent en évidence l'activité anti-microbienne de l'association selon l'invention, celle-ci étant comparée à l'activité anti-microbienne d'un lipoaminoacide : le N, n-octanoyl-glycine, vendu et commercialisé par SEPPIC sous la dénomination Lipacid ® C8G, et un monoalkyltéher de glycérol : le Sensiva® SC 50.

### Mode opératoire

Souches utilisées :
Staphylococcus aureus (ATCC 6538)
Escherichia coli (ATCC 8739)
Pseudomonas aeruginosa (ATCC 9027)
Enterococcus faecalis (ATCC 11700)
Candida albicans (ATCC 10231)

Des dilutions du produit à étudier sont réalisées en tubes dans le bouillon tryptone-sel à raison de 10ml par tube.

L'inoculum est constitué d'une culture de 24 heures du germe-test dans le bouillon nutritif tryptone-sel et dilué de façon à obtenir une suspension contenant 10⁵ à 10⁶ germes/ml. L'inoculum est ajouté sous un volume égal (10ml) aux différentes dilutions du produit ainsi que dans un tube témoin contenant 10 ml de bouillon tryptone-sel.

Un dénombrement est immédiatement effectué à partir du bouillon de ce tube témoin par ensemencement de dilutions décimales de ce bouillon sur mileu gélosé.

Tous les tubes sont incubés en atmosphère aérobie à 37°C pendant 24 heures.

Lecture : la CMI est la concentration minimale de produit pour laquelle il n'y a plus de culture visible. Un dénombrement des germes survivants est fait par repiquage sur milieu gélosé des bouillons des tubes restés sans culture visible.

Les résultats de CMI sont rassemblés dans les tableaux suivants.
- Tableau 1 :: CMI du Lipacid® C8G
- Tableau 2 :: CMI de l'association Lipacid® C8G/Sensiva® SC 50
- Tableau 3 :: CMI de Sensiva® SC 50
- + :: pas d'activité anti-microbienne détectée
- - :: activité anti-microbienne détectée

**Tableau 1**

| Concentration en % | E. coli | Pseudo. aeroginosa | Staph. aureus | Enter. faecalis | Candida albicans |
|---|---|---|---|---|---|
| 3 | - | - | - | - | - |
| 2 | - | - | - | - | - |
| 1 | - | - | - | - | - |
| 0,5 | - | - | - | - | - |
| 0,25 | - | - | - | - | - |
| 0,2 | - | - | - | - | - |
| 0,15 | - | - | - | - | - |
| 0,1 | - | - | + | - | + |
| 0,05 | + | + | + | + | + |
| 0,01 | + | + | + | + | + |

**Tableau 2**

| Concentration en % Lip./Sens | E. coli | Pseudo. aeroginosa | Staph. aureus | Enter. faecalis | Candida albicans |
|---|---|---|---|---|---|
| 0,5/0,5 | - | - | - | - | - |
| 0,1/0,1 | - | - | - | - | - |
| 0,1/0,05 | - | - | - | - | + |
| 0,05/0,1 | - | - | - | - | - |
| 0,05/0,05 | - | - | - | + | + |

**Tableau 3**

| Concentration en % | E. coli | Pseudo. aeroginosa | Staph. aureus | Enter. faecalis | Candida albicans |
|---|---|---|---|---|---|
| 3 | - | + | - | - | - |
| 2 | - | + | - | - | - |
| 1 | - | + | - | - | - |
| 0,5 | - | + | - | - | - |
| 0,25 | - | + | - | - | - |
| 0,2 | - | + | - | - | - |
| 0,15 | - | + | - | - | - |
| 0,1 | - | + | + | + | + |
| 0,05 | + | + | + | + | + |

Sensiva® SC 50 vendu par les laboratoires Phacogène est le composé : 1-(2-ethylhexyl)glycéroléther. Il est connu comme n'ayant pas d'activité anti-microbienne propre.

Ainsi, de manière tout à fait surprenante, le Sensiva® SC 50 à faible,voire sans activité anti-microbienne, a un effet de synergie sur l'activité anti-microbienne du Lipacid® C8G et plus particulièrement sur la bactérie gram - : Pseudomonas aeruginosa.

### EXEMPLE 2

On a préparé ici une composition utilisable en tant que crème de soin pour la peau et qui se présente sous la forme d'une émulison huile dans eau.

Il est préparé les mélanges suivants.

### Mélange A :

| | |
|---|---|
| Stéarate d'isocétyle | 9,0 % |
| Squalane | 7,0 % |
| Mélange monostéarate de glycérol/stéarate de polyéthylèneglycol (Arlacel® 165 de ICI) | 4,0 % |
| Alcool cétylique | 3,0 % |
| Amidon de mais estérifié par l'anhydre octényl succinique, sel d'aluminium (Dry-Flo Plus® -28-1160- de National Starch) | 2,0 % |
| Beurre de karité | 2,0 % |
| 1-(2-éthylhexyl)glycéroléther (Sensiva® SC50) | 0,5 % |

### Mélange B :

| | |
|---|---|
| Eau | qsp 100 % |
| N,n-octanoyl-glycine (Lipacid® C8G) | 0,5 % |
| Glycérol | 6,0 % |

### Mélange C :

| | |
|---|---|
| Mélange de (13/87) polydiméthylsiloxane α,ω dihydroxyle/cyclotétra et cyclopenta diméthyl siloxane (56% et 44%) | 3,0 % |
| Cyclométhicone | 7,0 % |

On porte le mélange A à 70°C sous agitation. On porte le mélange B à 70°C sous agitation. On transfère rapidement et sous vide le mélange B dans le mélange A, puis on émulsionne pendant 5 minutes. On refroidit à 45°C et on ajoute le mélange C sous agitation. On refroidit l'ensemble à 25°C.

## Revendications

1. Composition à usage topique, caractérisée en ce qu'elle comprend au moins un composé à activité anti-microbienne choisi parmi des hydrolipides, qui sont des produits résultant du couplage d'acides gras avec des molécules hydrophiles choisies parmi les acides aminés et les polysaccharides, et des lipides, qui sont des acides gras ramifiés ou non, saturés ou insaturés, de 6 à 18, de préférence de 6 à 12 atomes de carbone ou qui sont choisis parmi l'acide myristique, myristoléique, palmitique, palmitoléique, oléique, linoléique et linolénique, et au moins un monoalkyléther de glycérol.

2. Composition selon la revendication 1, caractérisée en ce que le composé à activité anti-microbienne est un hydrolipide, et plus particulièrement un lipoaminoacide.

3. Composition selon la revendication 1 ou 2, caractérisée en ce que les acides gras présents dans les hydrolipides présentent de 6 à 18, de préférence de 6 à 12, atomes de carbone.

4. Composition selon l'une quelconque des revendications précédentes, caractérisée en ce que lorsque les hydrolipides comprennent des acides aminés, ces derniers sont sous forme de monomères, de peptides ou de protéines, de préférence sous forme de monomères.

5. Composition selon la revendication précédente, caractérisée en ce que les acides aminés sont la glycine ou la cystéïne.

6. Composition selon l'une quelconque des revendications précédentes, caractérisée en ce que les monoalkyléthers de glycérol utilisés présentent la formule (I) suivante:
R-O-CH₂-CHOH-CH₂OH (I)
dans laquelle R représente un radical alkyle linéaire ou ramifié, saturé ou insaturé, de 3 à 14 atomes de carbone, en particulier de 5 à 12 atomes de carbone, de préférence de 7 à 9 atomes de carbone.

7. Composition selon la revendication précédente, caractérisée en ce que les monoalkyléthers de glycérol sont choisis parmi le 1-heptylglycéroléther, le 1-(2-éthylhexyl)glycéroléther, le 1-octylglycéroléther, le 1-décylglycéroléther et le 1-dodécylglycéroléther.

8. Composition selon l'une quelconque des revendications précédentes, caractérisée en ce que le rapport pondéral du composé à activité anti-microbienne par rapport au monoalkyléther de glycérol est compris entre 0,05 et 30, plus particulièrement entre 0,1 et 5.

9. Composition selon l'une quelconque des revendications précédentes, caractérisée en ce qu'elle est destinée à un usage cosmétique ou pharmaceutique, de préférence dermatologique.

10. Composition selon l'une quelconque des revendications précédentes, caractérisée en ce qu'elle comprend de 0,01 à 6 %, avantageusement de 0,1 à 2 %, en poids de monoalkyléther de glycérol par rapport au poids total de la composition.

11. Composition selon l'une quelconque des revendications précédentes, caractérisée en ce qu'elle comprend de 0,01 à 5 %, avantageusement de 0,2 à 3 %, en poids de composé à activité anti-microbienne par rapport au poids total de la composition.

12. Utilisation de la composition selon l'une quelconque des revendications 1 à 11 en tant qu'agent anti-microbien dans une composition cosmétique ou pharmaceutique.

13. Utilisation de la composition selon l'une quelconque des revendications 1 à 11 dans la préparation d'une composition pharmaceutique anti-microbienne, plus particulièrement dermatologique.

## Claims

1. Composition for topical use, characterized in that it comprises at least one compound with antimicrobial activity chosen from hydrolipids, which are products resulting from the coupling of fatty acids with hydrophilic molecules chosen from amino acids and polysaccharides, and lipids, which are branched or unbranched, saturated or unsaturated fatty acids of 6 to 18 carbon atoms, preferably of 6 to 12 carbon atoms, or which are chosen from myristic acid, myristoleic acid, palmitic acid, palmitoleic acid, oleic acid, linoleic acid and linolenic acid, and at least one monoalkyl glyceryl ether.

2. Composition according to Claim 1, characterized in that the compound with antimicrobial activity is a hydrolipid and more particularly a lipoamino acid.

3. Composition according to Claim 1 or 2, characterized in that the fatty acids present in the hydrolipids have from 6 to 18 and preferably from 6 to 12 carbon atoms.

4. Composition according to any one of the preceding claims, characterized in that when the hydrolipids comprise amino acids, the latter are in the form of monomers, peptides or proteins, preferably in the form of monomers.

5. Composition according to the preceding claim, characterized in that the amino acids are glycine or cysteine.

6. Composition according to any one of the preceding claims, characterized in that the glyceryl monoalkyl ethers used have the following formula (I):
R-O-CH₂-CHOH-CH₂OH (I)
in which R represents a linear or branched, saturated or unsaturated alkyl radical of 3 to 14 carbon atoms, in particular of 5 to 12 carbon atoms, preferably of 7 to 9 carbon atoms.

7. Composition according to the preceding claim, characterized in that the glyceryl monoalkyl ethers are chosen from 1-heptyl glyceryl ether, 1-(2-ethylhexyl) glyceryl ether, 1-octyl glyceryl ether, 1-decyl glyceryl ether and 1-dodecyl glyceryl ether.

8. Composition according to any one of the preceding claims, characterized in that the weight ratio of the compound with antimicrobial activity to the glyceryl monoalkyl ether is between 0.05 and 30 and more particularly between 0.1 and 5.

9. Composition according to any one of the preceding claims, characterized in that it is intended for cosmetic or pharmaceutical use, preferably for dermatological use.

10. Composition according to any one of the preceding claims, characterized in that it comprises from 0.01 to 6%, advantageously from 0.1 to 2%, by weight of glyceryl monoalkyl ether relative to the total weight of the composition.

11. Composition according to any one of the preceding claims, characterized in that it comprises from 0.01 to 5%, advantageously from 0.2 to 3%, by weight of compound with antimicrobial activity relative to the total weight of the composition.

12. Use of the composition according to any one of Claims 1 to 11 as antimicrobial agent in a cosmetic or pharmaceutical composition.

13. Use of the composition according to any one of Claims 1 to 11 in the preparation of an antimicrobial pharmaceutical composition, more particularly a dermatological composition.

## Patentansprüche

1. Zusammensetzung zur topischen Anwendung, dadurch **gekennzeichnet,** daß sie mindestens eine Verbindung mit antimikrobieller Wirkung, die unter den Hydrolipiden, die Produkte sind, die sich aus der Bindung von Fettsäuren mit hydrophilen Molekülen ergeben, welche unter den Aminosäuren und den Polysacchariden ausgewählt sind, und den Lipiden ausgewählt ist, welche verzweigte oder unverzweigte, gesättigte oder ungesättigte Fettsäuren mit 6 bis 18 und vorzugsweise 6 bis 12 Kohlenstoffatomen sind oder welche unter Myristinsäure, Myristoleinsäure, Palmitinsäure, Palmitoleinsäure, Ölsäure, Linolsäure und Linolensäure ausgewählt sind, und mindestens einen Glycerinmonoalkylether enthält.

2. Zusammensetzung nach Anspruch 1, dadurch **gekennzeichnet,** daß die Verbindung mit antimikrobieller Wirkung ein Hydrolipid und insbesondere Lipoaminosäure ist.

3. Zusammensetzung nach Anspruch 1 oder 2, dadurch **gekennzeichnet,** daß die in den Hydrolipiden vorliegenden Fettsäuren 6 bis 18 und vorzugsweise 6 bis 12 Kohlenstoffatome aufweisen.

4. Zusammensetzung nach einem der vorhergehenden Ansprüche, dadurch **gekennzeichnet,** daß, falls die Hydrolipide Aminosäuren enthalten, diese in Form von Monomeren, Peptiden oder Proteinen und vorzugsweise in Form von Monomeren vorliegen.

5. Zusammensetzung nach dem vorhergehenden Anspruch, dadurch **gekennzeichnet,** daß es sich bei den Aminosäuren um Glycin oder Cystein handelt.

6. Zusammensetzung nach einem der vorhergehenden Ansprüche, dadurch **gekennzeichnet,** daß die verwendeten Glycerinmonoalkylether die folgende Formel (I) aufweisen:
R-O-CH₂-CHOH-CH₂-OH (I)
worin R eine geradkettige oder verzweigte, gesättigte oder ungesättigte Alkylgruppe mit 3 bis 14, insbesondere 5 bis 12 und vorzugsweise 7 bis 9 Kohlenstoffatomen bedeutet.

7. Zusammensetzung nach dem vorhergehenden Anspruch, dadurch **gekennzeichnet,** daß die Glycerinmonoalkylether unter 1-Heptylglycerinether, 1-(2-Ethylhexyl)glycerinether, 1-Octylglycerinether, 1-Decylglycerinether und 1-Dodecylglycerinether ausgewählt sind.

8. Zusammensetzung nach einem der vorhergehenden Ansprüche, dadurch **gekennzeichnet,** daß das Gewichtsverhältnis der Verbindung mit antimikrobieller Wirkung zum Glycerinmonoalkylether im Bereich von 0,05 bis 30 und insbesondere im Bereich von 0,1 bis 5 liegt.

9. Zusammensetzung nach einem der vorhergehenden Ansprüche, dadurch **gekennzeichnet,** daß sie für eine kosmetische oder pharmazeutische und vorzugsweise dermatologische Verwendung bestimmt ist.

10. Zusammensetzung nach einem der vorhergehenden Ansprüche, dadurch **gekennzeichnet,** daß sie 0,01 bis 6 Gew.-% und vorteilhaft 0,1 bis 2 Gew.-% Glycerinmonoalkylether, bezogen auf das Gesamtgewicht der Zusammensetzung, enthält.

11. Zusammensetzung nach einem der vorhergehenden Ansprüche, dadurch **gekennzeichnet,** daß sie 0,01 bis 5 Gew.-% und vorteilhaft 0,2 bis 3 Gew.-% der Verbindung mit antimikrobieller Wirkung, bezogen auf das Gesamtgewicht der Zusammensetzung, enthält.

12. Verwendung der Zusammensetzung nach einem der Ansprüche 1 bis 11 als antimikrobielles Mittel in einer kosmetischen oder pharmazeutischen Zusammensetzung.

13. Verwendung der Zusammensetzung nach einem der Ansprüche 1 bis 11 bei der Herstellung einer pharmazeutischen und insbesondere dermatologischen, antimikrobiellen Zusammensetzung.
